# EUROPEAN PATENT SPECIFICATION

(11) **EP 1 740 148 B1**
(45) Date of publication and mention of the grant of the patent: **13.10.2010**
(21) Application number: 05725768.5
(22) Date of filing: 16.03.2005
(51) Int. Cl.: A61K 8/894, A61Q 15/00, C11D 3/18

(54) **LOW MOLECULAR WEIGHT SILICONE OIL-IN-WATER EMULSION**
NIEDERMOLEKULARE ÖL-IN-WASSER-SILIKONEMULSION
EMULSION AQUEUSE DE SILICONE DE FAIBLE MASSE MOLECULAIRE

(30) Priority: 15.04.2004 US 562658 P
(43) Date of publication of application: 10.01.2007
(73) Proprietor: Dow Corning Corporation, Midland, Michigan 48686 (US)
(72) Inventor: FENG, Qian, Jane, Midland, MI 48640 (US); HICKERSON, Robin, Sue, Beaverton, MI 48612 (US); STARCH, Michael, Stephen, Midland, MI 48642 (US); VAN REETH, Isabelle, B-1315 Incourt (BE)
(74) Representative: Polypatent
(86) International application number: PCT/US2005/008811
(87) International publication number: WO 2005/105024

(56) References cited:
- WO-A-2005/065630
- WO-A1-2005/065630
- US-A- 5 783 535
- US-A- 5 783 535
- US-A- 5 853 711
- US-A- 5 853 711
- US-A- 5 879 671
- US-B1- 6 652 867

## Description

### Cross Reference to Related Applications

None.

### Technical Field

This invention provides oil in water emulsion compositions, and methods for preparing such emulsions, where the oil phase component of the emulsion contains at least 50 weight percent of a low molecular weight siloxane. The oil in water emulsions are stabilized by a combination of a silicone polyether and anionic surfactant. The oil in water emulsions, and subsequently diluted emulsions therefrom, are stable with time and are useful in a variety of personal, medical, and household care formulations.

### Background

Emulsions of silicones are well known in the art and many are commercially available. However, oil in water emulsions of low molecular weight silicones, or volatile siloxanes, are not as common due to their inherent instability. More specifically, oil in water emulsions where majority of the oil phase is composed of a volatile siloxane, are difficult to stabilize because the average particle size of the dispersed oil phase increases with time due to a process known as Ostwald Ripening. Low molecular weight siloxanes have sufficient solubility in water such that they can diffuse from one oil phase particle to another. Thus with time, the low molecular weight siloxanes can diffuse from smaller particles to larger ones in an emulsion, causing an increase in the average particle size, and eventually destabilizing the emulsion.

There is a particular need and interest to stabilize low molecular weight silicone oil as the main oil phase component in water continuous emulsions for applications in the personal, medical, and household care industries. Low molecular weight or volatile methyl siloxanes are commonly used in many formulated consumer products such as antiperspirants, deodorants, cosmetics, shampoos, conditioners, skin care lotions and creams. Stable water based emulsions of low molecular weight siloxanes are of interest to formulators of such consumer products because of the increased formulating latitude offered by a water dilutable product. Thus, there is a need for silicone oil in water emulsion where the silicone oil is primarily a volatile siloxane that is stable with time. Moreover, there is a need for such emulsions that have dilution stability, that is, can be diluted or formulated with other ingredients while maintaining its emulsion particle integrity. This is especially needed for incorporating volatile methyl siloxanes in highly ionic formulations, such as antiperspirant salt formulations. Emulsions of this type also are expected to have utility in the preparation of non-woven articles, such as "wipes" type products. The emulsions can be applied (for example by spraying) on to a non-woven fabric sheet, for preparation of cleansing products for both household and medical care products.

Some examples of oil in water emulsions, where the oil phase is mainly composed of low molecular weight siloxanes include; U.S. Patent Nos. 2,755,194, 4,784,844 and 5,300,286. However, these references do not disclose any long-term stability of the reported emulsions, nor specifically disclose compositions or processes for making emulsions with long term stability.

U.S. 5,443,760 by Kasprzak teaches silicone containing oil in water emulsions prepared with certain siloxane-oxyalkylene copopolymeric surfactants. The oil phase includes silicone oils which are volatile and silicone oils or gums which are non-volatile, or a mixture of silicone oils and gums. Kasprzak teaches the use of at least two siloxane-oxyalkylene copolymeric surfactants, one in the oil phase and the other in the aqueous phase, and the combination of both surfactants must have a combined HLB value of 4 - 7.

More recently, US 6,087,317 by Gee discloses particle size stable silicone emulsions of low molecular weight siloxanes. Gee's emulsions are stabilized by using a nonionic surfactant as the primary emulsifier having an hydrophilic-lipophilic balance (HLB) greater than about 13, at a concentration sufficient to provide about 0.5-3 molecules per 100 square Å of surface area of silicone particles; and a nonionic cosurfactant having an HLB less than about 11, at a concentration sufficient to provide 5-15 molecules of emulsifier per 100 square Å of surface area of silicone particles.

WO 03/011948 discloses clear microemulsions by simply combining (i) water; (ii) a volatile siloxane; (iii) a long chain or high molecular weight silicone polyether; and, as an optional ingredient, (iv) a cosurfactant such as a monohydroxy alcohol, an organic diol, an organic triol, an organic tetraol, a silicone diol, a silicone triol, a silicone tetraol, and a nonionic organic surfactant.

WO 03/064500 discloses compositions containing silicone in oil water emulsions, salts, alcohols, and solvents. The silicone in oil emulsions of the WO 03/064500 publication are prepared by polymerizing silicon atom containing monomers in water containing the monomer, a silicone polyether, a catalyst, and optionally an organic surfactant(s), until a silicone oil of a desired molecular weight is obtained.

US-A-5 879 671 (HALLORAN ET AL) 9 March 1999 (1999-03-09) discloses a personal care product (Table I) comprising an emulsion comprising:
b- a trisiloxane polyether
c- ammonium lauryl sulfate as an anionic surfactant
d- water.

While these references represent an advance in the state of the art, a need still exists for volatile silicone in water emulsions that can be diluted, or alternatively can be formulated with other ingredients, and yet maintain emulsion particle integrity. More so, there is a need for volatile silicone in water emulsions that can be formulated into antiperspirant salt compositions that are storage stable.

The present inventors have discovered storage stable and dilution stable low molecular weight silicone in water emulsion compositions that fulfill the needs as described above. The emulsions were unexpectedly realized when certain silicone polyethers and anionic co-surfactants were used in combination to emulsify volatile silicones.

### Summary

The present invention provides an emulsion composition comprising:
A) 20 to 80 weight % of an oil phase containing at least 50 weight % of a low molecular weight siloxane;
B) a silicone polyether having the formula 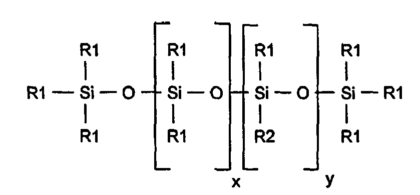 or 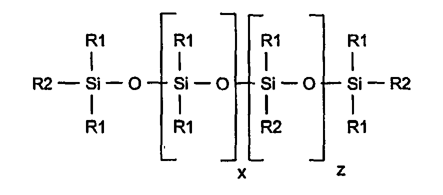 where R1 represents an alkyl group containing 1-6 carbon atoms;
R2 represents the radical -(CH₂)ₐO(C₂H₄O)_{b}(C₃H₆O)_{c}R3;
x is 20-1,000; y is 2-500; z is 2-500; a is 3-6; b is 4-30; c is 0-30; and R3 is hydrogen, a methyl radical, or an acyl radical;
C) an anionic surfactant; and
D) water in a sufficient amount such that the sum of the weight percents of A), B), C), and D) equals 100 weight percent,
wherein the oil phase is dispersed in the emulsion as particles having an average size of less than 5 micrometers.

The present invention also relates to a method of making an oil in water emulsion comprising:
I) mixing;
   A) a low molecular weight siloxane, and
   B) a silicone polyether having the formula 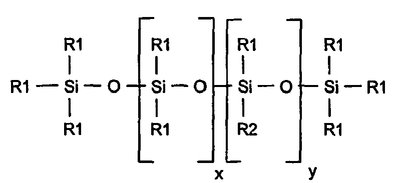 or 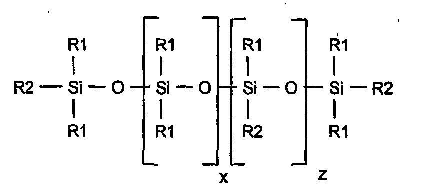 where R1 represents an alkyl group containing 1-6 carbon atoms;
      R2 represents the radical -(CH₂)ₐO(C₂H₄O)_{b}(C₃H₆O)_{c}R3;
      x is 20-1,000; y is 2-500; z is 2-500; a is 3-6; b is 4-30; c is 0-30; and R3 is hydrogen, a methyl radical, or an acyl radical;
      to form an oil phase wherein the oil phase contains at least 50% by weight of the low molecular weight siloxane, and
II) adding the oil phase to an aqueous phase comprising;
   C) an anionic surfactant, and
   D) water,
   to form an oil in water emulsion,
   wherein the emulsion contains 20-80 weight percent of the oil phase.

The present invention also relates to the use of the emulsion of claim 1 to prepare a personal care product.

The compositions and products produced by the methods of the present invention are useful in a variety of applications where stable and dilutable water based emulsions of low molecular weight silicones are desired. In particular, the present low molecular weight silicone emulsions are useful in antiperspirant compositions due to their enhanced formulation stability. The emulsions of the present invention are useful in various sprayable lotions and wipe applications.

### Detailed Description

Component (A) in the present invention is an oil phase containing at least 50% of a low molecular weight siloxane. As used herein, the phrase low molecular weight siloxane is intended to mean and to include polysiloxanes having the general formula RᵢSiO_{(4-i)/2} in which i has an average value of one to three, and having a molecular weight (M_{w}) of less than 1000, R is any monovalent organic group, but typically R is a methyl group. Alternatively, the structures of the low molecular weight siloxanes can be represented by monofunctional "M" units (CH₃)₃SiO_{1/2} , difunctional "D" units (CH₃)₂SiO_{2/} ,trifunctional "T" units, and tetrafunctional "Q" units SiO_{4/2}. The phrase "low molecular weight siloxane" is intended to mean and to include (i) low molecular weight linear and cyclic volatile methyl siloxanes, (ii) low molecular weight functional linear and cyclic siloxanes. Most preferred, however, are the low molecular weight linear and cyclic volatile methyl siloxanes (VMS). Volatile methyl siloxanes conforming to the CTFA definition of cyclomethicones are also considered to be within the definition of low molecular weight siloxane.

Linear VMS have the formula (CH₃)₃SiO{(CH₃)₂SiO}_{f}Si(CH₃)₃. The value of f is 0-7. Cyclic VMS have the formula {(CH₃)₂SiO}_{g}. The value of g is 3-6. Preferably, these volatile methyl siloxanes have a molecular weight of less than about 1,000; a boiling point less than about 250°C; and a viscosity of about 0.65 to about 5.0 centistoke (mm²/s), generally not greater than 5.0 centistoke (mm²/s).

Representative linear volatile methyl siloxanes are hexamethyldisiloxane (MM) with a boiling point of 100 °C, viscosity of 0.65 mm²/s, and formula Me₃SiOSiMe₃; octamethyltrisiloxane (MDM) with a boiling point of 152 °C, viscosity of 1.04 mm²/s, and formula Me₃SiOMe₂SiOSiMe₃; decamethyltetrasiloxane (MD₂M) with a boiling point of 194 °C, viscosity of 1.53 mm²/s, and formula Me₃SiO(Me₂SiO)₂SiMe₃; dodecamethylpentasiloxane (MD₃M) with a boiling point of 229 °C, viscosity of 2.06 mm²/s, and formula Me₃SiO(Me₂SiO)₃SiMe₃; tetradecamethylhexasiloxane (MD₄M) with a boiling point of 245 °C, viscosity of 2.63 mm²/s, and formula Me₃SiO(Me₂SiO)₄SiMe₃; and hexadecamethylheptasiloxane (MD₅M) with a boiling point of 270 °C, viscosity of 3.24 mm²/s, and formula Me₃SiO(Me₂SiO)₅SiMe₃.

Representative cyclic volatile methyl siloxanes are hexamethylcyclotrisiloxane (D₃), a solid with a boiling point of 134 °C, a molecular weight of about 223, and formula {(Me₂)SiO}₃; octamethylcyclotetrasiloxane (D₄) with a boiling point of 176 °C, viscosity of 2.3 mm²/s, a molecular weight of about 297, and formula {(Me₂)SiO}₄; decamethylcyclopentasiloxane (D₅) with a boiling point of 210 °C, viscosity of 3.87 mm²/s, a molecular weight of about 371, and formula {(Me₂)SiO}₅; and dodecamethylcyclohexasiloxane (D₆) with a boiling point of 245 °C, viscosity of 6.62 mm²/s, a molecular weight of about 445, and formula {(Me₂)SiO}₆.

Representative branched volatile methyl siloxanes are heptamethyl-3-{(trimethylsilyl)oxy}trisiloxane (M₃T) with a boiling point of 192 °C, viscosity of 1.57 mm²/s, and formula C₁₀H₃₀O₃Si₄; hexamethyl-3,3,bis {(trimethylsilyl)oxy} trisiloxane (M₄Q) with a boiling point of 222 °C, viscosity of 2.86 mm²/s, and formula C₁₂H₃₆O₄Si₅; and pentamethyl {(trimethylsilyl)oxy} cyclotrisiloxane (MD₃) with the formula C₈H₂₄O₄Si₄.

The invention also includes using low molecular weight linear and cyclic volatile and non-volatile higher alkyl and aryl siloxanes, represented respectively by formulas R₃SiO(R₂SiO)_{f}SiR₃ and (R₂SiO)_{g}. R can be alkyl groups with 2-20 carbon atoms or aryl groups such as phenyl. The value of f is 0 to about 7. The value of g is 3-6. These values should be selected to provide polysiloxanes with a viscosity generally not greater than about 5 centistoke (mm²/s), and with a molecular weight of less than about 1,000. Illustrative of such polysiloxanes are polydiethylsiloxane, polymethylethylsiloxane, polymethylphenylsiloxane, and polydiphenylsiloxane.

Low molecular weight functional polysiloxanes can also be employed, and are represented by the formula R₃SiO(RQSiO)_{f}SiR₃ or the formula (RQSiO)g where Q is a functional group. Examples of such functional polysiloxanes are acrylamide functional siloxane fluids, acrylate functional siloxane fluids, amide functional siloxane fluids, amino functional siloxane fluids, carbinol functional siloxane fluids, carboxy functional siloxane fluids, chloroalkyl functional siloxane fluids, epoxy functional siloxane fluids, glycol functional siloxane fluids, ketal functional siloxane fluids, mercapto functional siloxane fluids, methyl ester functional siloxane fluids, perfluoro functional siloxane fluids, silanol functional siloxanes, and vinyl functional siloxane fluids. Again, the values of f and g, and the functional group Q, are selected to provide functional polysiloxanes with a viscosity generally not greater than about 5 centistoke (mm²/s), and a molecular weight of less than about 1,000.

Providing that at least 50 weight percent of the oil phase contains a low molecular weight siloxane, alternatively 60 weight percent of a low molecular weight siloxane alternatively 70 weight percent of a low molecular weight siloxane, or alternatively 80 weight percent, the oil phase may contain other components that are dispersible in the selected low molecular weight siloxane. These other oil phase components can be selected from any silicone, hydrocarbon, or personal care active that is substantially soluble in the low molecular weight siloxane, and conversely, is substantially insoluble in water. Thus, other typical oil phase components can include, high molecular weight (i.e. M_{w} > 1000) siloxanes, including silicone elastomers and resins, hydrocarbon oils, waxes, emollients, fragrances, and personal care organic actives such as vitamins and sunscreens. Typically, when the other oil phase component is primarily a polar oil such as a vegetable oil, it is preferred that at least 50 weight percent of the oil phase contain a low molecular weight siloxane.

High molecular weight siloxanes can be added to the oil phase having the formula RᵢSiO_{(4-i)/2} in which i has an average value of one to three, R is a monovalent organic group. The phrase high molecular weight means a molecular weight (M_{w}) of equal to or greater than 1000. Thus, the high molecular weight siloxane can be selected from any polydiorganosiloxanes fluids or gum having a molecular weight equal to or greater than 1000. The polydiorganosiloxane gums suitable for the present invention are essentially composed of dimethylsiloxane units with the other units being represented by monomethylsiloxane, trimethylsiloxane, methylvinylsiloxane, methylethylsiloxane, diethylsiloxane, methylphenylsiloxane, diphenylsiloxane, ethylphenylsiloxane, vinylethylsiloxane, phenylvinylsiloxane, 3,3,3-trifluoropropylmethylsiloxane, dimethylphenylsiloxane, methylphenylvinylsiloxane, dimethylethylsiloxane, 3,3,3-trifluoropropyldimethylsiloxane, mono-3,3,3-trifluoropropylsiloxane, aminoalkylsiloxane, monophenylsiloxane, monovinylsiloxane and the like. The polydiorganosiloxane gums are well known in the art and can be obtained commercially, and which have viscosities greater than 1,000,000 cs. at 25.degree. C., preferably greater than 5,000,000 cs. at 25.degree. C.

Suitable oil components include, but are not limited to, natural oils such as coconut oil; hydrocarbons such as mineral oil and hydrogenated polyisobutene; fatty alcohols such as octyldodecanol; esters such as C12 -C15 alkyl benzoate; diesters such as propylene dipelarganate; and triesters, such as glyceryl trioctanoate. The other oil phase components can also be mixture of low viscosity and high viscosity oils. Suitable low viscosity oils have a viscosity of 5 to 100 mPa.s at 25°C, and are generally esters having the structure RCO-OR' wherein RCO represents the carboxylic acid radical and wherein OR' is an alcohol residue. Examples of these low viscosity oils include isotridecyl isononanoate, PEG-4 diheptanoate, isostearyl neopentanoate, tridecyl neopentanoate, cetyl octanoate, cetyl palmitate, cetyl ricinoleate, cetyl stearate, cetyl myristate, coco-dicaprylate/caprate, decyl isostearate, isodecyl oleate, isodecyl neopentanoate, isohexyl neopentanoate, octyl palmitate, dioctyl malate, tridecyl octanoate, myristyl myristate, octododecanol, or mixtures of octyldodecanol, acetylated lanolin alcohol, cetyl acetate, isododecanol, polyglyceryl-3-diisostearate, or mixtures thereof. The high viscosity surface oils generally have a viscosity of 200-1,000,000 mPa.s at 25°C, preferably a viscosity of 100,000-250,000 mPa.s. Surface oils include castor oil, lanolin and lanolin derivatives, triisocetyl citrate, sorbitan sesquioleate, C10-18 triglycerides, caprylic/capric/triglycerides, coconut oil, corn oil, cottonseed oil, glyceryl triacetyl hydroxystearate, glyceryl triacetyl ricinoleate, glyceryl trioctanoate, hydrogenated castor oil, linseed oil, mink oil, olive oil, palm oil, illipe butter, rapeseed oil, soybean oil, sunflower seed oil, tallow, tricaprin, trihydroxystearin, triisostearin, trilaurin, trilinolein, trimyristin, triolein, tripalmitin, tristearin, walnut oil, wheat germ oil, cholesterol, or mixtures thereof. Mention may be made, among the optional other non-silicone fatty substances, of mineral oils, such as liquid paraffin or liquid petroleum, of animal oils, such as perhydrosqualene or arara oil, or alternatively of vegatable oils, such as sweet almond, calophyllum, palm, castor, avocado, jojaba, olive or cereal germ oil. It is also possible to use esters of lanolic acid, of oleic acid, of lauric acid, of stearic acid or of myristic acid, for example; alcohols, such as oleyl alcohol, linoleyl or linolenyl alcohol, isostearyl alcohol or octyldodecanol; or acetylglycerides, octanoates, decanoates or ricinoleates of alcohols or of polyalcohols. It is alternatively possible to use hydrogenated oils which are solid at 25°C, such as hydrogenated castor, palm or coconut oils, or hydrogenated tallow; mono-, di-, tri- or sucroglycerides; lanolins; or fatty esters which are solid at 25°C.

The common assignee's U.S. Patent 5,948,855 (September 7, 1999), also contains an extensive list of some appropriate oil soluble active ingredients such as vitamins and drugs which can be used in the oil phase of the oil in water emulsions, among which are vitamins, including but not limited to, Vitamin A₁, RETINOL, C₂-C₁₈ esters of RETINOL, Vitamin E, TOCOPHEROL, esters of Vitamin E, and mixtures thereof. RETINOL includes trans-RETINOL, 13-cis-RETINOL, 11-cis-RETINOL, 9-cis-RETINOL, and 3,4-didehydro-RETINOL. Other vitamins which are appropriate include RETINYL ACETATE, RETINYL PALMITATE, RETINYL PROPIONATE, α-TOCOPHEROL, TOCOPHERSOLAN, TOCOPHERYL ACETATE, TOCOPHERYL LINOLEATE, TOCOPHERYL NICOTINATE, and TOCOPHERYL SUCCINATE.

The compositions of the present invention contains 20 to 80, alternatively 20 to 60, or alternatively 20 to 40 weight % of the oil phase, component (A).

Component (B) is a silicone polyether having the structure represented by: 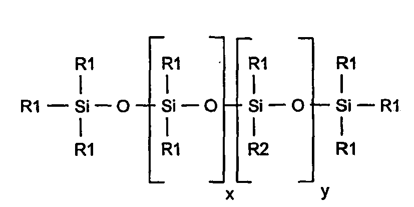 or

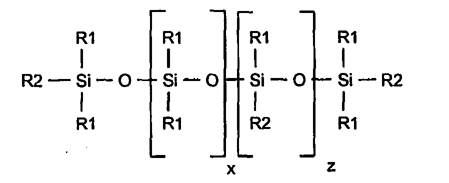

In these structures, R1 represents an alkyl group containing 1-6 carbon atoms such as methyl, ethyl, propyl, butyl, pentyl, and hexyl; R2 represents the radical - (CH₂)ₐO(C₂H₄O)_{b}(C₃H₆O)_{c}R3; x has a value of 20-1,000, alternatively 20-200, or alternatively 20-50; y has a value of 2-500, alternatively 2-50, or alternatively 2-10, z has a value of 2-500, alternatively 2-50, or alternatively 2-10; m has a value of 3-5; n is one; a has a value of 3-6; b has a value of 4-30; c has a value of 0-30; and R3 is hydrogen, a methyl radical, or an acyl radical such as acetyl. Preferably, R1 is methyl; b is 6-25; c is zero; and R3 is hydrogen.

Typically, the silicone polyether is chosen such that the ratio of x/y or x/z, as described in the structures above, ranges from 2:1 to 50:1, alternatively from 5:1 to 20:1, or alternatively from 10:1 to 12:1.

The silicone polyether can be prepared by any of the techniques known in the art, and many are commercially available.

The compositions of the present invention contains 2 to 20, alternatively 2 to 15, or alternatively 5 to 10 weight % of the silicone polyether, component (B).

Component (C) is an anionic surfactant. Any anionic surfactant known in the art to stabilize oil in water emulsions can be selected as component (C). Examples of suitable anionic surfactants include alkali metal sulforicinates, sulfonated glyceryl esters of fatty acids such as sulfonated monoglycerides of coconut oil acids, salts of sulfonated monovalent alcohol esters such as sodium oleylisothianate, amides of amino sulfonic acids such as the sodium salt of oleyl methyl tauride, sulfonated products of fatty acids nitriles such as palmitonitrile sulfonate, sulfonated aromatic hydrocarbons such as sodium alpha-naphthalene monosulfonate, condensation products of naphthalene sulfonic acids with formaldehyde, sodium octahydroanthracene sulfonate, alkali metal alkyl sulfates such as sodium lauryl sulfate, ammonium lauryl sulfate or triethanol amine lauryl sulfate, ether sulfates having alkyl groups of 8 or more carbon atoms such as sodium lauryl ether sulfate, ammonium lauryl ether sulfate, sodium alkyl aryl ether sulfates, and ammonium alkyl aryl ether sulfates, alkylarylsulfonates having 1 or more alkyl groups of 8 or more carbon atoms, alkylbenzenesulfonic acid alkali metal salts exemplified by hexylbenzenesulfonic acid sodium salt, octylbenzenesulfonic acid sodium salt, decylbenzenesulfonic acid sodium salt, dodecylbenzenesulfonic acid sodium salt, cetylbenzenesulfonic acid sodium salt, and myristylbenzenesulfonic acid sodium salt, sulfuric esters of polyoxyethylene alkyl ether including CH₃(CH₂)₆CH₂O(C₂H₄O)₂SO₃H, CH₃(CH₂)₇CH₂O(C₂H₄O)_{3.5}SO₃H, CH₃(CH₂)₈CH₂O(C₂H₄O)₈SO₃H, CH₃(CH₂)₁₉CH₂O(C₂H₄O)₄SO₃H, and CH₃(CH₂)₁₀CH₂O(C₂H₄O)₆SO₃H, sodium salts, potassium salts, and amine salts of alkylnaphthylsulfonic acid.

Alternatively, the anionic surfactant is selected from a sulfate of ethoxylated alcohols. Sulfates of ethoxylated alcohols are well known in the art and many are sold commercially under numerous tradenames such as ALFONINIC, NEODOL, STANDAPOL ES, STEOL, SULFOTEX, TEXAPON, WICOLATE. Alternatively, the anionic surfactant is STANDAPOL ES or EMPICOL ESB-3.

The compositions of the present invention contains 0.1 to 2, alternatively 0.1 to 1, or alternatively .2 to .5 weight % of the anionic surfactant, component (C).

Preferably, the oil in water emulsion compositions contain concentrations of the silicone polyether and the anionic surfactant such that the weight ratio of the silicone polyether to anionic surfactant is 5 to 60, alternatively 10 to 40, or alternatively 10 to 30.

The emulsion compositions of the present invention can be prepared by any technique known in the art for preparing oil in water emulsions. Typically, the emulsions are prepared by mixing components (A), (B), (C) and (D) using mixing techniques such as milling, blending, homogenizing, sonolating or stirring. These mixing procedures can be conducted either in a batch or continuous process.

Alternatively, the compositions of the present invention can be prepared by the methods described infra.

The oil in water emulsion compositions of the present invention comprise an oil phase, component (A), dispersed in a water continuous phase. The oil phase is stabilized in the emulsion by the combination of the silicone polyether, (B), and anionic surfactant, (C). The oil phase is thus dispersed in the water continuous phase as discrete oil phase particles. Typically, the oil phase particles have an average particle size that is less than 5 micrometers, alternatively less than 1 micrometers, or alternatively less than 0.5 micrometers. "Average particle size" is the accepted meaning in the emulsion art, and can be determined for example using a particle size analyzer such as a *Nanotrac 150.*

The oil in water emulsions can be considered as stable water continuous emulsions. For purposes of this invention, "stable water continuous emulsion" means that the emulsion's average particle size distribution does not change substantially within a given period of time. Stability can be evaluated by storing samples of the emulsion at room temperature for a period of time and observing the emulsions for any signs of instability, such as separation or creaming. Samples can also be stored at elevated temperatures, for example 50°C, to simulate an accelerate aging process. Typically, the emulsions of the present invention do not show separation at room temperature after aging for at least 4 weeks. Typically the average particle size remains less than 5 µm particle size for at least 4 weeks at room temperature, and the change in average particle size is < 10 % after 4 week at 50°C.

In a preferred embodiment, herein referred to as dilution stable oil in water emulsion composition, the amounts of components (A), (B), (C), and (D) are selected to provide an oil in water emulsion that yields stable water continuous emulsions upon further aqueous dilution. In particular, the dilution stable oil in water emulsion comprises;
A) 20 to 80 weight % of an oil phase containing at least 50% of a low molecular weight siloxane;
B) 2 to 20 weight % of a silicone polyether having the formula 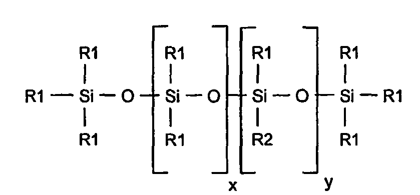 or 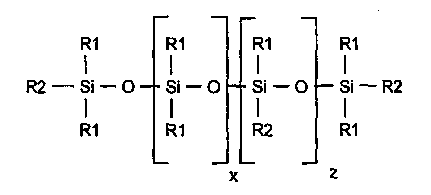 where R1 represents an alkyl group containing 1-6 carbon atoms;
   R2 represents the radical -(CH₂)ₐO(C₂H₄O)_{b}(C₃H₆O)_{c}R3;
   x is 20-1,000; y is 2-500; z is 2-500; a is 3-6; b is 4-30; c is 0-30; and R3 is hydrogen, a methyl radical, or an acyl radical;
C) 0.1 to 2 weight % of an anionic surfactant; and
D) sufficient water to sum to 100 weight percent.

Other optional ingredients may be added to the oil in water emulsions of the present invention as desired to affect certain performance properties, providing the nature and/or quantity of these optional ingredients does not substantially destabilize the emulsions of the present invention. These optional ingredients include, fillers, freeze-thaw additives such as ethylene glycol or propylene glycol, antimicrobial preparations, UV filters, pigments, dyes, and perfumes. These optional ingredients also include thickeners and stabilizers known in the art to stabilize or thicken oil in water emulsions, such as cellulose based thickeners, polyvinyl alcohols, polyacrylic acids or carbomers.

The present invention also relates to a method for preparing an oil in water emulsion comprising:
I) mixing;
   A') a low molecular weight siloxane, and
   B) a silicone polyether having the formula 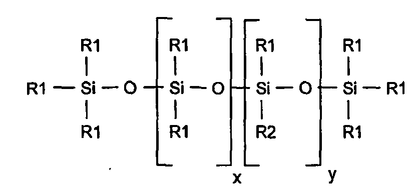 or 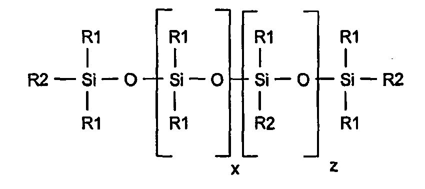 where R1 represents an alkyl group containing 1-6 carbon atoms;
      R2 represents the radical -(CH₂)ₐO(C₂H₄O)_{b}(C₃H₆O)_{c}R3;
      x is 20-1,000; y is 2-500; z is 2-500; a is 3-6; b is 4-30; c is 0-30; and R3 is hydrogen, a methyl radical, or an acyl radical;
      to form an oil phase wherein the oil phase contains at least 50% by weight of the low molecular weight siloxane, and
II) adding the oil phase to an aqueous phase comprising;
   C) an anionic surfactant, and
   D) water,
   to form an oil in water emulsion,
   wherein the emulsion contains 20-80 weight percent of the oil phase.

For step I) of the present method, the low molecular weight siloxane A') and B) the silicone polyether are the same as described supra. Additional components can be added to the low molecular weight siloxane, also described supra, when forming the oil phase, providing that the low molecular weight siloxane comprises at least 50 weight percent of the oil phase. Mixing can be performed by any conventional known techniques such as milling, blending, homogenizing, sonolating or stirring. These mixing procedures can be conducted either in a batch or continuous process.

Step II) of the present method involves adding the oil phase prepared in step I) to an aqueous phase comprising an anionic surfactant and water. The anionic surfactant is the same as described supra. The oil phase is typically added to the aqueous phase with mixing or shearing so as to form a oil in water emulsion by any mixing technique known in the art for preparing oil in water emulsions. These mixing procedures can be conducted either in a batch or continuous process.

The emulsion prepared according to the invention can be used in various over-the-counter (OTC) personal care products, medical care, and household care products. Thus, they can be used in antiperspirants, deodorants, skin creams, skin care lotions, moisturizers, facial treatments such as acne or wrinkle removers, personal and facial cleansers, bath oils, perfumes, colognes, sachets, sunscreens, pre-shave and after-shave lotions, liquid soaps, shower gels, shaving soaps, shaving lathers, hair shampoos, hair conditioners, hair sprays, mousses, permanents, depilatories, cuticle coats, make-ups, color cosmetics, foundations, blushes, lipsticks, lip balms, eyeliners, mascaras, oil removers, color cosmetic removers, and powders. Furthermore, it is anticipated that the emulsion compositions of the present invention can be combined with various other components to prepare the personal care products described infra. These components include additional surfactants, moisturizers, pigments, sunscreens, fragrances, emollients, commonly used to formulate such personal care products. The emulsion compositions of the present invention can also be used in sprayable lotions formulations and in various wipe formulations.

### EXAMPLES

The following examples are presented to further illustrate the compositions and methods of this invention, but are not to be construed as limiting the invention, which is delineated in the appended claims. All parts and percentages in the examples are on a weight basis and all measurements were obtained at about 23°C, unless indicated to the contrary.

### Materials

The following describes the silicone polyether used in the examples.
SPE 1 = a silicone polyether composition containing > 90 wt % of a silicone polyether having the average formula of MD₂₂D^{R}₂M, where M, D, and D^{R} represent the (CH₃)₃SiO_{1/2}, (CH₃)₂SiO , and (CH₃)RSiO siloxy units respectively, and R = -CH₂CH₂CH₂O(CH₂CH₂O)₁₂H. The silicone polyether composition results from the platinum catalyzed hydrosilylation reaction of MD₂₂D₂^{H} M (where D^{H} represents the (CH₃)HSiO siloxy unit) with a slight molar excess of H₂C=CHCH₂O(CH₂CH₂O)₁₂H, according to known procedures, such as those taught in US Patent No. 4,147,847.

### Example 1

### D5 emulsion with SPE 1 and SLES

Part A: in a small beaker, 8 g Dow Corning® 245 Fluid and 3.4g SPE 1 were premixed.
Part B: in a cream jar, 28.46g deionized water and 0.14g Standapol ES-3 (sodium lauryl ether sulfate) were combined and mixed.

The contents of part A were poured into part B. The mixture was sonicated with a sonic probe in pulsed mode for 2 minutes. The resulting emulsion had a particle size of 239 nanometers. The particle size of this emulsion did not change after 20 days at 50°C. When 0.5 g of this emulsion was diluted into 10 g water, the particle size of the resulting emulsion did not change after 7 days at room temperature. When 2 g of an aluminum chloride solution (50%) was added to 2 grams of this emulsion and shaken, the resulting composition was stable, no separation was observed.

### Example 2 (Comparative example)

### D5 emulsion with SLES

In a cream jar, 31.2g deionized water and 0.8g Standapol ES-3 (sodium lauryl ether sulfate) were premixed. Then, 8 g Dow Corning® 245 Fluid was added. The mixture was sonicated with a soniprobe in pulsed mode for 2 minutes. The resulting emulsion had an initial particle size of 327 nanometers, but separated after 5 days in 50°C. When 2 g of an aluminum chloride solution (50%) was added to 2 grams of this emulsion and mixed, the resulting emulsion was not stable as evidenced by breaking into different layers instantly.

### Example 3 (Comparative Example)

### D5 emulsion with SPE 1

Part A: in a beaker, 8 g Dow Corning® 245 Fluid and 3.4g SPE 1 were premixed.
Part B: in a cream jar, 28.46g deionized water was loaded.

The contents of part A were poured into part B. The mixture was sonicated with a soniprobe in pulsed mode for 2 minutes. The resulting emulsion had a particle size of 329 nanometers. An oil layer appeared at the top of the emulsion after 1 day at room temperature indicating instability of the emulsion.

### Example 4

### D5/100 cst PDMS emulsion with SPE 1 and SLES

Part A: in a beaker, 75.1 g Dow Corning® 245 Fluid, 25 g Dow Corning® 100 cst Fluid and 42.17 g SPE 1 were premixed.
Part B: in another beaker, 355.95 g deionized water and 1.78 g Standapol ES-3 (sodium lauryl ether sulfate) were combined and mixed.

The contents of part A were poured slowly into part B with continuous mixing with a lab mixer agitating at 1350 RPM. The final mixture was agitated at 1350 RPM for an additional 30 minutes at room temperature. The mixture was further treated with a Silverson high shear mixer for 2-3 minutes to produce an emulsion. When 2 g of an aluminum chloride solution (50%) was added to this emulsion and mixed, the resulting emulsion was stable without separation.

### Example 5

An emulsion was prepared using the same procedure as in Example IV, but replacing Dow Corning® 100cst Fluid with an equal amount in weight of Dow Corning® 556 Fluid by the resulted in an emulsion, hereafter referred to as Emulsion V.

Aluminum chloride solution (50%) in the amount of 2 gram was added to 2 grams of Emulsion V and mixed and shaken. The composition was stable.

### Example 6

An emulsion was prepared using the same procedure as Example I except the Dow Corning® 245 Fluid was replaced by an equal amount in weight of Dow Corning® 1184 Fluid (mixture of volatile silicone fluids including linear and cyclics). The resulting emulsion was stable and remained stable even after mixing 2 g of a 50% aluminum chloride solution.

### Example 7

A series of emulsions were prepared having an oil phase containing a mixture of an emollient oil or sunscreen active with a volatile siloxane, the formulations of which are summarized in Table 1. The emulsions for these examples contained 40 wt % of the oil phase components and 15 weight % of SPE 1 based on the total weight of the emulsion formulation. These emulsions were prepared by first mixing the volatile siloxane with the additional oil phase component, as listed in examples VII a - f in Table 1, for 10 minutes via a lab mixer (200 rpm). Separately, an aqueous phase was prepared by mixing the aqueous phase components, as listed in examples VII a-f in Table 1, for 10 minutes via a lab mixer (200 rpm). Then, the oil phase was slowly added to the aqueous phase with mixing via a lab mixer (900 rpm). Following complete addition of the oil phase to the aqueous phase, mixing was continued for an additional 30 minutes at a mixing speed of 900 RPM. Finally, the resulting composition was passed through a Silverson mixer (Model SL2). All the emulsions were considered to be stable as no visible separation was observed at room temperature for 1 months and one month at 40°C.

### Example 8

A series of emulsions were prepared having an oil phase containing a mixture of an emollient oil or sunscreen active with a volatile siloxane, the formulations of which are summarized in Table 2. The emulsions for these examples contained 20 weight % of the oil phase components and 8.5 weight % of SPE 1 based on the total weight of the emulsion formulation. These emulsions were prepared by the procedure described in Example 7. These emulsions were stable, as evidenced by no separation after storing at room temperature and 40°C for one month.

### Example 9

An oil in water emulsion was prepared according to the procedure of Example 8 which contained 8.5% SPE 1, 20 wt % DC245 as phase A, and 0.34 wt % Empicol ESB-3, and 71.16 wt% water. The resulting emulsion was impregnated onto a nonwoven fabric and its ability to perform as a wipe product for the removal of a medical adhesive on skin. This wipe product containing the emulsion performed had equivalent cleansing ability as compared to a commercial wipe.

### Example 10

An oil in water emulsion was prepared according to the procedure of Example 1 which contained 8.5% SPE 1 and 20% DC 245 as part A, and 0.35% Empicol ESB-3 and 71.15% water. The resulting emulsion was then mixed with *REACH 301,* an antiperspirant salt solution containing 50% activated aluminum sesquichlorohydrate in various ratios, as summarized in Table 3. The resulting formulations were stable, that is, it did not show any signs of separation at room temperature for one month and less than 10% creaming after one month at 45°C.

### Example 11

An oil in water emulsion was prepared according to the procedure of Example 6 which contained 8.5% SPE 1 and 20% DC 1184 as part A, and 0.35% Empicol ESB-3 and 71.15% water. The resulting emulsion was then mixed with *REACH 301,* an antiperspirant salt solution containing 50% activated aluminum sesquichlorohydrate in various ratios, as summarized in Table 3. The resulting formulations were stable, that is did not show any signs of separation at room temperature for one month and less than 10% creaming after one month at 45°C.

**Table 1**

| | wt % | wt % | wt % | wt % | wt % | wt % | wt % | wt % | wt % |
|---|---|---|---|---|---|---|---|---|---|
| **Oil phase** | | | | | | | | | |
| SPE 1 | 15 | 15 | 15 | 15 | 15 | 15 | 15 | 15 | 15 |
| DC245 | 20 | 30 | 20 | 30 | 30 | 35 | 30 | 30 | 30 |
| Mineral oil | | 10 | | | | | | | |
| isododecane | 20 | | | | | | | | |
| Isohexadecane | | | 20 | | | | | | |
| Crodamol AB | | | | | | | | | |
| (Tegasoft TN) | | | | 10 | | | | | |
| Crodamol GTCC | | | | | 10 | | | | |
| Castor oil | | | | | | 5 | | | |
| Eutanol G | | | | | | | 10 | | |
| Crodamol OP | | | | | | | | 10 | |
| Parsol MCX | | | | | | | | | 7 |
| Parsol 1789 | | | | | | | | | 3 |
| | | | | | | | | | |
| **Water phase** | | | | | | | | | |
| Empicol ESB-3 | 0.75 | 0.75 | 0.75 | 0.75 | 0.75 | 0.75 | 0.75 | 0.75 | 0.75 |
| Water | to 100% | to 100% | to 100% | to 100% | to 100% | to 100% | to 100% | to 100% | to 100% |

**Table 2**

| | | Wt % | wt% | wt% | wt% | wt% |
|---|---|---|---|---|---|---|
| Oil phase | SPE 1 | 8.5 | 8.5 | 8.5 | 8.5 | |
| | DC245 | 15 | 10 | 15 | 15 | 15 |
| | Mineral oil | 5 | | | | |
| | Isohexadecane | | 10 | | | |
| | Crodamol AB (Tegasoft TN) | | | 5 | | |
| | Crodamol GTCC | | | | 5 | |
| | Petrolatum | | | | | 5 |
| | | | | | | |
| Water phase | | | | | | |
| | Empicol ESB-3 | 0.34 | 0.34 | 0.34 | 0.34 | 0.34 |
| | Water | to 100% | to 100% | to 100% | to 100% | to 100% |

**Table 3**

| | wt% | wt% | wt% | wt% | wt% | wt% | wt% | wt% |
|---|---|---|---|---|---|---|---|---|
| Example 10 emulsion | 50 | | 50 | | 25 | | 25 | |
| Example 11 emulsion | | 50 | | 50 | | 25 | | 25 |
| AP salt | 40 | 40 | 20 | 20 | 10 | 10 | 40 | 40 |
| water | 10 | 10 | 30 | 30 | 65 | 65 | 35 | 35 |
| % actives | 20 | 20 | 10 | 10 | 5 | 5 | 20 | 20 |
| % volatile Silicone | 10 | 10 | 10 | 10 | 5 | 5 | 5 | 5 |

### Example 12

### Emulsion of D5/DC 2502 AMS at 1:1 ratio with SPE I and SLES

An emulsion was prepared according to the procedure of example 1 having the following components;

Part A: in a small beaker, 4 g Dow Corning® 245 Fluid, 4 g Dow Corning® 2502 AMS and 2.6 g SPE 1 were premixed.
Part B: in a cream jar, 29.26 g deionized water and 0.14 g Standapol ES-3 (sodium lauryl ether sulfate) were combined and mixed.

The resulting emulsion had a particle size of 201 nanometers. The particle size increased 6% to 0.213 nanometers after heated at 50°C for 20 days.

### Example 13

### Emulsion of D5/DC 2502 AMS at 1:1 ratio with SPE 1 and SLES

An emulsion was prepared according to the procedure of example 1 having the following components;

Part A: in a small beaker, 4 g Dow Corning® 245 Fluid, 4 g Dow Corning® 50 cst fluid and 2.6 g SPE 1 were premixed.
Part B: in a cream jar, 29.26 g deionized water and 0.14 g Standapol ES-3 (sodium lauryl ether sulfate) were combined and mixed.

The resulting emulsion had a particle size of 205 nanometers and did not change at 50°C for 20 days.

### Example 14

### Comparative example

The following emulsion was prepared according to the procedures described in US 6,087,317 by R. Gee.
In a cream jar, 23.48 g deionized water and 1.28 g SPAN 20 and 1.32 Tween 80 were combined and mixed. Then, 14 g of Dow Corning® 245 Fluid were poured into the jar. The mixture was sonicated with a soniprobe in pulsed mode for 2 minutes. The resulting emulsion had a particle size of 300 nanometers. When 2 g of an aluminum chloride solution (50%) was added to 2 grams of this emulsion and mixed, the resulting emulsion was not stable as evidenced by separation into layers and becoming yellowish after heated for 3 days at 50°C.

### Example 15

### Shampoo containing emulsion of low molecular weight siloxane

Shampoo compositions containing a representative emulsion of the present invention were prepared according to the following procedure and formulation;
1. mix phase A ingredients together and heat to 60°C,
2. add phase B while mixing and heating,
3. let cool to room temperature,
4. add phase F,
5. adjust pH to 6 with phase D

| | **Ingredients** | | |
|---|---|---|---|
| | | | |
| *Phase A* | | | |
| | Empicol ESB3 | 30.0% | 30.0% |
| | Water | to 100% | to 100% |
| | Glucamate DOE120 | 1.5% | 1.5% |
| | Rewoderm S1333 | 4.0% | 4.0% |
| | Amonyl 380BA | 4.0% | 4.0% |
| *Phase B* | | | |
| | Comperlan KD | 4.0% | 4.0% |
| *Phase D* | | | |
| | Citric acid (50%) to pH=6 | q.s. | q.s. |
| *Phase F* | Emulsion of 20 wt% D5* | 10 | |
| | Emulsion of 40wt% D5** | | 5 |
| | | | |

| | | | |
|---|---|---|---|
| * 20 wt % of D5, 8.5 % of SPE 1 and 0.35 % of Empicol ESB 3, prepared using a procedure similar as example 1. ** 40 wt % of D5, 8.5 % of SPE 1 and 0.35 % of Empicol ESB 3, prepared using a procedure similar in example 1 | | | |

### Example 16

### Rinse off conditioner containing emulsion of low molecular weight siloxane

Rinse off conditioning compositions containing a representative emulsion of the present invention were prepared according to the following procedure and formulation;
1) Prepare Part A by sifting the HEC into the water while stirring rapidly.
After all the HEC is dispersed, mix in the Arquad. Stir the dispersion until the HEC goes into solution. When the HEC goes into solution, it will thicken and look translucent.
(if you leave Part A stand so that the air bubbles come out, it is clear).
2) Combine the ingredients for Part B in a small beaker and heat to 80-85°C
3) Heat Part C to 80-85°C in the final beaker
4) Mix Part B and then add rapidly to Part C with rapid stirring. Mix for 5-10 minutes
5) Add Part A (at room temperature) to the hot emulsion.
Add Part A over a period of 2-3 minutes so as not to cool the batch down too quickly.
Increase the mixer speed so as to maintain good turnover as the batch thickens. Continue mixing until the batch cools to 40-45°C.
6) Compensate water loss and add phase E
7) Adjust pH to 4 with phase D

| | **Ingredients** | | | |
|---|---|---|---|---|
| | | | | |
| *Phase A* | | | | |
| | Natrosol 250HHR | Hydroxyethyl cellulose | 1.5% | 1.5% |
| | Arquad 16-29 | Cetrimonium chloride (29%) | 0.3% | 0.3% |
| | Water | | 50.0% | 50.0% |
| *Phase B* | | | | |
| | Cetyl alcohol | | 1.0% | 1.0% |
| | Arlacel 165 | PEG-100 stearate& Glyceryl stearate | 1.0% | 1.0% |
| | | | | |
| | | | | |
| *Phase C* | | | | |
| | Water | | 36.2% | 41.2% |
| *Phase D* | | | | |
| | Citric acid | | q.s. | q.s. |
| Phase E | Emulsion of D5* | | 10.0% | |
| | Emulsion of D5* | | | 5.0% |

| | | | | |
|---|---|---|---|---|
| * 20 wt % of D5, 8.5 % of SPE 1 and 0.35 % of Empicol ESB 3, prepared using a procedure similar as example 1. ** 40 wt % of D5, 8.5 % of SPE 1 and 0.35 % of Empicol ESB 3, prepared using a procedure similar in example 1 | | | | |

## Claims

1. An oil in water emulsion composition comprising:
A) 20 to 80 weight % of an oil phase containing at least 50 weight % of a low molecular weight siloxane having a molecular weight (M_{w}) of less than 1000;
B) a silicone polyether having the formula 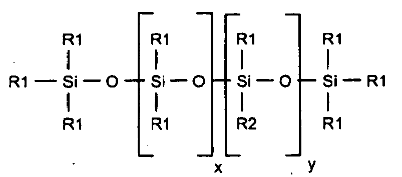 or 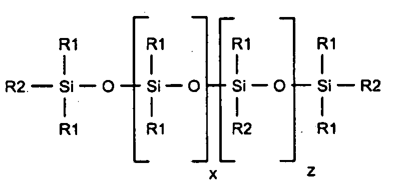 where R1 represents an alkyl group containing 1-6 carbon atoms; R2 represents the radical -(CH₂)ₐO(C₂H₄O)_{b}(C₃H₆O)_{c}R3;
x is 20-1,000; y is 2-500; z is 2-500; a is 3-6; b is 4-30; c is 0-30; and R3 is hydrogen, a methyl radical, or an acyl radical;
C) an anionic surfactant; and
D) water in a sufficient amount such that the sum of the weight percents of A), B), C), and D) equals 100 weight percent,
wherein the oil phase is dispersed in the emulsion as particles having an average size of less than 5 micrometers.

2. The emulsion of claim 1 wherein the emulsion comprises:
20 - 80 weight % A) oil phase,
2- 20 weight % B) silicone polyether,
0.1-2 weight % C) anionic surfactant,
a sufficient amount of D) water such that the sum of the weight percents of A), B), C), and D) equals 100 weight percent.

3. The emulsion of claim 1 wherein the low molecular weight siloxane is selected from the group of hexamethyldisiloxane, octamethyltrisiloxane, decamethyltetrasiloxane, dodecamethylpentasiloxane, tetradecamethylhexasiloxane, hexadecamethylheptasiloxane, hexamethylcyclotrisiloxane, octamethylcyclotetrasiloxane, decamethylcyclopentasiloxane, dodecamethylcyclohexasiloxane, and mixtures thereof.

4. The emulsion of claim 1 wherein the oil phase further comprises a silicone oil, hydrocarbon oil, or natural oil.

5. The emulsion of claim 1 wherein the ratio x/y or x/z of the silicone polyether ranges from 2:1 to 50:1.

6. The emulsion of claim 1 wherein the anionic surfactant is a sulfate of an ethoxylated alcohol.

7. The emulsion of claim 1 further comprising a filler, freeze-thaw additive, antimicrobial agent, UV filter, pigment, dye, perfume, and mixtures thereof.

8. A method for preparing an oil in water emulsion comprising:
I) mixing;
A) a low molecular weight siloxane having a molecular weight (M_{w}) of less than 1000, and
B) a silicone polyether having the formula 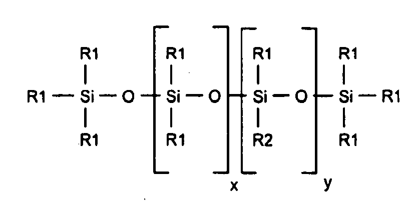 or 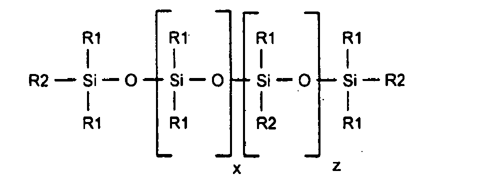 where R1 represents an alkyl group containing 1-6 carbon atoms; R2 represents the radical -(CH₂)ₐO(C₂H₄O)_{b}(C₃H₆O)_{c}R3; x is 20-1,000; y is 2-500; z is 2-500; a is 3-6; b is 4-30; c is 0-30; and R3 is hydrogen, a methyl radical, or an acyl radical;
to form an oil phase wherein the oil phase contains at least 50% by weight of the low molecular weight siloxane, and
II) adding the oil phase to an aqueous phase comprising:
C) an anionic surfactant, and
D) water,
to form an oil in water emulsion, wherein the emulsion contains 20-80 weight percent of the oil phase.

9. Use of the emulsion of claim 1 to prepare a personal care product.

10. Use of claim 9 wherein the personal care product is selected from antiperspirants, deodorants, skin creams, skin care lotions, moisturizers, facial treatments such as acne or wrinkle removers, personal and facial cleansers, bath oils, perfumes, colognes, sachets, sunscreens, pre-shave and after-shave lotions, liquid soaps, shower gels, shaving soaps, shaving lathers, hair shampoos, hair conditioners, hair sprays, mousses, permanents, depilatories, cuticle coats, make-ups, color cosmetics, foundations, blushes, lipsticks, lip balms, eyeliners, mascaras, oil removers, color cosmetic removers, and powders.

11. A non-woven fabric coated with the emulsion of claim 1.

## Patentansprüche

1. Öl-in-Wasser-Emulsionszusammensetzung enthaltend:
A) 20-80 Gew.-% einer Ölphase, die wenigstens 50 Gew.-% eines Siloxans mit niedrigem Molekulargewicht mit einem Molekulargewicht (M_{w}) von weniger als 1000 enthält,
B) einen Siliconpolyether mit der Formel 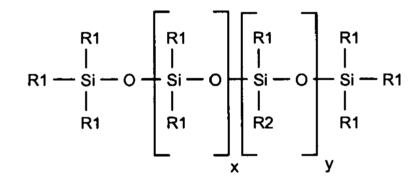 oder 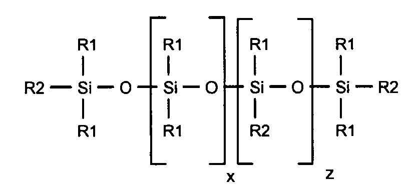 worin R1 für eine Alkylgruppe mit 1-6 Kohlenstoffatomen steht, R2 für den Rest -(CH₂)ₐO(C₂H₄O)_{b}(C₃H₆O)_{c}R3 steht,
x gleich 20-1.000 ist, y gleich 2-500 ist, z gleich 2-500 ist, a gleich 3-6 ist, b gleich 4-30 ist, c gleich 0-30 ist und R3 gleich Wasserstoff, ein Methylrest oder ein Acylrest ist,
C) ein anionisches oberflächenaktives Mittel und
D) Wasser in einer ausreichenden Menge, so dass die Summe der Gewichtsprozentanteile von A), B), C) und D) gleich 100 Gew.-% ist,
wobei die Ölphase in der Emulsion als Teilchen mit einer mittleren Größe von weniger als 5 µm dispergiert ist.

2. Emulsion nach Anspruch 1, wobei die Emulsion enthält:
20-80 Gew.-% A) Ölphase,
2-20 Gew.-% B) Siliconpolyether,
0,1-2 Gew.-% C) anionisches oberflächenaktives Mittel,
eine ausreichende Menge an D) Wasser, so dass die Summe der Gewichtsprozentanteile von A), B), C) und D) gleich 100 Gew.-% ist.

3. Emulsion nach Anspruch 1, wobei das Siloxan mit niedrigem Molekulargewicht ausgewählt ist aus der Gruppe aus Hexamethyldisiloxan, Octamethyltrisiloxan, Decamethyltetrasiloxan, Dodecamethylpentasiloxan, Tetradecamethylhexasiloxan, Hexadecamethylheptasiloxan, Hexamethylcyclotrisiloxan, Octamethylcyclotetrasiloxan, Decamethylcyclopentasiloxan, Dodecamethylcyclohexasiloxan und Mischungen davon.

4. Emulsion nach Anspruch 1, wobei die Ölphase weiterhin ein Siliconöl, Kohlenwasserstofföl oder ein natürliches Öl enthält.

5. Emulsion nach Anspruch 1, wobei das Verhältnis x/y oder x/z des Siliconpolyethers von 2:1 bis 50:1 reicht.

6. Emulsion nach Anspruch 1, wobei das anionische oberflächenaktive Mittel ein Sulfat eines ethoxylierten Alkohols ist.

7. Emulsion nach Anspruch 1, die zusätzlich einen Füllstoff, ein Gefrier-Tau-Additiv, ein antimikrobielles Mittel, einen UV-Filter, ein Pigment, einen Farbstoff, ein Parfum oder Mischungen davon enthält.

8. Verfahren zur Herstellung einer Öl-in-Wasser-Emulsion umfassend:
I) Mischen von
A) einem Siloxan mit niedrigem Molekulargewicht, das ein Molekulargewicht (M_{w}) von weniger als 1000 aufweist, und
B) einem Siliconpolyether mit der Formel 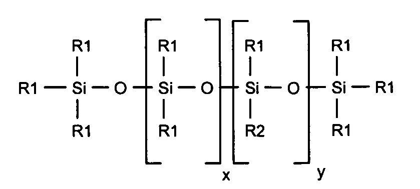 oder 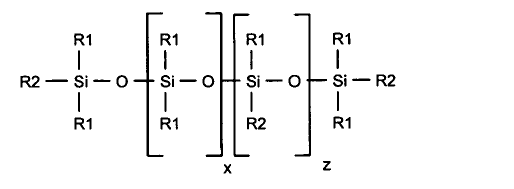 worin R1 für eine Alkylgruppe mit 1-6 Kohlenstoffatomen steht, R2 für den Rest -(CH₂)ₐO(C₂H₄O)_{b}(C₃H₆O)_{c}R3 steht, x gleich 20-1.000 ist, y gleich 2-500 ist, z gleich 2-500 ist, a gleich 3-6 ist, b gleich 4-30 ist, c gleich 0-30 ist und R3 gleich Wasserstoff, ein Methylrest oder ein Acylrest ist,
um eine Ölphase zu bilden, wobei die Ölphase wenigstens 50 Gew.-% des Siloxans mit niedrigem Molekulargewicht enthält, und II) Zugeben der Ölphase zu einer wässrigen Phase, die
C) ein anionisches oberflächenaktives Mittel und
D) Wasser enthält,
um eine Öl-in-Wasser-Emulsion zu bilden, wobei die Emulsion 20-80 Gew.-% der Ölphase enthält.

9. Verwendung der Emulsion nach Anspruch 1, um ein Körperpflegeprodukt herzustellen.

10. Verwendung nach Anspruch 9, wobei das Körperpflegeprodukt ausgewählt ist aus Antitranspirationsmitteln, Deodorantien, Hautcremes, Hautpflegelotionen, feuchtigkeitsspendenden Präparaten, Gesichtsbehandlungspräparaten wie Anti-Akne- oder Anti-Faltenpräparaten, Körper- und Gesichtsreinigungsmitteln, Badeölen, Parfums, Eau de Colognes, Sachets, Sonnenschutzmitteln, Pre-Shave- und After-Shave-Lotionen, Flüssigseifen, Duschgelen, Rasierseifen, Rasierschäumen, Haarshampoos, Haarkonditionierern, Haarsprays, Schäumen, Dauerwellenpräparaten, Haarentfernungsmitteln, Nagelhautabdeckern, Make-Ups, dekorativen Kosmetika, Grundierungen, Rouges, Lippenstiften, Lippenbalsamen, Eyelinern, Wimperntuschen, Ölentfernern, Make-Up-Entfernern und Pudern.

11. Nichtgewebtes Textil, das mit der Emulsion nach Anspruch 1 beschichtet ist.

## Revendications

1. Composition d'émulsion huile-dans-eau comprenant :
A) 20 à 80 % en poids d'une phase huileuse contenant au moins 50 % en poids d'un siloxane de faible masse moléculaire ayant une masse moléculaire (Mₚ) inférieure à 1 000 ;
B) un silicone polyéther ayant la formule 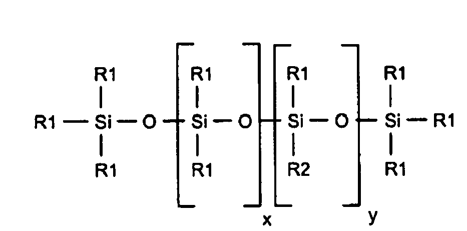 ou 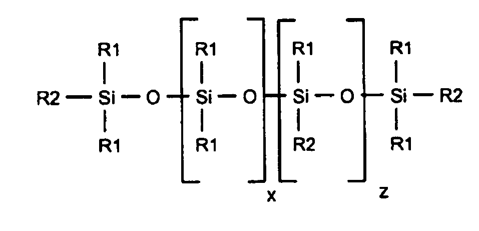 où R1 représente un groupe alkyle contenant 1-6 atomes de carbone ;
R2 représente le radical -(CH₂)ₐO(C₂H₄O)_{b}(C₃H₆O)_{c}R3 ;
x est 20-1 000 ; y est 2-500 ; z est 2-500 ; a est 3-6 ; b est 4-30 ; c est 0-30 ; et
R3 est l'hydrogène, un radical méthyle ou un radical acyle ;
C) un tensioactif anionique ; et
D) de l'eau en une quantité suffisante pour que la somme des pourcentages en poids de A), B), C) et D) soit égale à 100 % en poids,
où la phase huileuse est dispersée dans l'émulsion sous forme de particules ayant une taille moyenne inférieure à 5 µm.

2. Emulsion selon la revendication 1 où l'émulsion comprend :
20-80 % en poids de A) phase huileuse,
2-20 % en poids de B) silicone polyéther,
0,1-2 % en poids de C) tensioactif anionique,
une quantité suffisante de D) eau pour que la somme des pourcentages en poids de A), B), C) et D) soit égale à 100 % en poids.

3. Emulsion selon la revendication 1 où le siloxane de faible masse moléculaire est choisi dans le groupe d'hexaméthyldisiloxane, d'octaméthyltrisiloxane, de décaméthyltétrasiloxane, de dodécaméthyl-pentasiloxane, de tétradécaméthylhexasiloxane, d'hexadécaméthyl-heptasiloxane, d'hexaméthylcyclotrisiloxane, d'octaméthylcyclotétrasiloxane, de décaméthylcyclopentasiloxane, de dodécaméthylcyclohexasiloxane, et leurs mélanges.

4. Emulsion selon la revendication 1 où la phase huileuse comprend en outre une huile de silicone, une huile hydrocarbonée ou une huile naturelle.

5. Emulsion selon la revendication 1 où le rapport x/y ou x/z du silicone polyéther va de 2:1 à 50:1.

6. Emulsion selon la revendication 1 où le tensioactif anionique est un sulfate d'un alcool éthoxylé.

7. Emulsion selon la revendication 1 comprenant en outre une charge, un additif de congélation-décongélation, un agent antimicrobien, un filtre UV, un pigment, un colorant, un parfum et leurs mélanges.

8. Procédé pour préparer une émulsion huile-dans-eau comprenant :
I) le mélange :
A) d'un siloxane de faible masse moléculaire ayant une masse moléculaire (Mₚ) inférieure à 1 000, et
B) d'un silicone polyéther ayant la formule 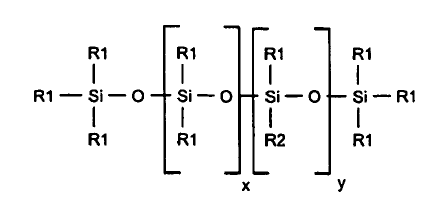 ou 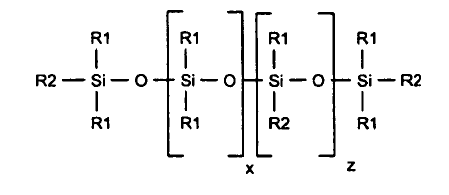 où R1 représente un groupe alkyle contenant 1-6 atomes de carbone ;
R2 représente le radical -(CH₂)ₐO(C₂H₄O)_{b}(C₃H₆O)_{c}R3 ;
x est 20-1 000 ; y est 2-500 ; z est 2-500 ; a est 3-6 ; b est 4-30 ; c est 0-30 ; et
R3 est l'hydrogène, un radical méthyle ou un radical acyle ;
pour former une phase huileuse où la phase huileuse contient au moins 50 % en poids du siloxane de faible masse moléculaire, et
II) l'addition de la phase huileuse à une phase aqueuse comprenant :
C) un tensioactif anionique, et
D) de l'eau,
pour former une émulsion huile-dans-eau où l'émulsion contient 20-80 % en poids de la phase huileuse.

9. Utilisation de l'émulsion selon la revendication 1 pour préparer un produit pour les soins personnels.

10. Utilisation selon la revendication 9 où le produit pour les soins personnels est choisi parmi les antisudoraux, les désodorisants, les crèmes pour la peau, les lotions pour les soins de la peau, les humectants, les traitements du visage comme les agents retirant l'acné ou les rides, les agents de nettoyage personnels et du visage, les huiles pour le bain, les parfums, les eaux de cologne, les sachets, les écrans solaires, les lotions d'avant-rasage et d'après-rasage, les savons liquides, les gels pour la douche, les savons à raser, les mousses à raser, les shampoings capillaires, les conditionneurs capillaires, les sprays capillaires, les mousses, les permanentes, les épilatoires, les revêtements pour l'épiderme, les maquillages, les cosmétiques colorés, les fonds de teint, les fards, les rouges à lèvres, les baumes pour les lèvres, les agents soulignant les yeux, les mascaras, les agents retirant les huiles, les agents retirant les cosmétiques colorés, et les poudres.

11. Etoffe non tissée revêtue de l'émulsion selon la revendication 1.
